# FASCICULE DE BREVET EUROPEEN

(11) **EP 2 364 682 B1**
(45) Date de publication et mention de la délivrance du brevet: **10.10.2012**
(21) Numéro de dépôt: 11154299.9
(22) Date de dépôt: 14.02.2011
(51) Int. Cl.: A61F 13/08

(54) **Orthèse compressive du membre inférieur de type bas ou collant, à apparence "collant 3/4"**
Kompressionsorthese der unteren Gliedmaßen vom Typ Strumpfhose oder Strümpfe in Form von 3/4 Strümpfen
Low or skin-tight orthosis for compressing the lower limbs, with a skin-tight 3/4 aspect

(30) Priorité: 09.03.2010 FR 1051684
(43) Date de publication de la demande: 14.09.2011
(73) Titulaire: Innothera Topic International, 94110 Arcueil (FR)
(72) Inventeur: Mathieu, M. Joël, 88510 Eloyes (FR); Rafstedt, M. Pauline, 54000 Nancy (FR); Cheny, M. Thierry, 54630 Richardmenil (FR)
(74) Mandataire: Dupuis-Latour, Dominique

(56) Documents cités:
- WO-A1-97/45081
- FR-A1- 2 780 637
- FR-A1- 2 877 726
- US-A- 4 086 790

## Description

L'invention concerne les orthèses de compression veineuse élastique (CVE), qui sont indiquées dans les diverses manifestations cliniques d'insuffisance veineuse des membres inférieurs.

Ces orthèses, anciennement connues sous la dénomination de "bas de contention" ou "collants de contention", sont des dispositifs médicaux textiles produisant un effet thérapeutique par compression/contention des membres inférieurs, par opposition aux "bas de maintien" (ou encore "bas de soutien" ou "bas anti-fatigue") et aux "bas mode", qui ne sont pas des dispositifs médicaux à visée thérapeutique.

Les orthèses de CVE sont conçues pour produire un effet thérapeutique par compression du membre inférieur sur une étendue plus ou moins grande, avec un profil dégressif vers le haut à partir de la cheville. Selon le type de bas, la pression mesurée à la cheville peut varier de 10 à plus de 36 mmHg (soit 13 à 48 hPa, le mmHg étant toutefois d'usage courant comme unité de mesure de pression dans le domaine de la phlébologie et de la compression médicale). Pour la France, les bas sont répartis selon le référentiel ASQUAL en quatre classes textiles, à savoir la classe I (13 à 20 hPa ≈ 10 à 15 mmHg à la cheville), la classe Il (20 à 27 hPa ≈ 15 à 20 mmHg), la classe III (27 à 48 hPa ≈ 20 à 36 mmHg) et la classe IV (> 48 hPa ≈ > 36 mmHg). Ces classes de compression peuvent être différentes pour d'autres pays.

Pour permettre une compression forte des membres inférieurs, ces bas médicaux sont réalisés à partir d'une maille tricotée de texture plus ou moins serrée avec incorporation d'un fil de trame élastique, généralement un élasthanne guipé.

Plus précisément, sous l'effet de la mise en place sur le membre, le textile tendu de l'orthèse exerce une compression résultant de la force de rappel des fibres élastiques qui composent le matériau, et l'application de ces forces de rappel élastique sur le périmètre du contour engendre en un point donné, selon la loi de Laplace, une pression locale inversement proportionnelle au rayon de courbure du contour en ce point.

Cette pression est la "pression textile" telle que définie et calculée au sens de la norme française NF G 30-102b. On désignera dans la présente description par "pression" la moyenne des pressions normalisées de contention/compression localement exercées à une altitude donnée le long d'un contour (contour circulaire ou elliptique, dans l'approximation d'une jambe-modèle).

La maille et les fils, ainsi que le dimensionnement des rangées de maille, sont choisis de manière à appliquer des pressions prédéterminées à différentes altitudes du membre inférieur, par exemple à la hauteur de la cheville, au départ du mollet, au niveau du mollet, au creux poplité, etc. jusqu'en haut de cuisse. Ces différentes pressions sont définies pour chaque classe de contention/compression en référence à des gabarits métrologiques tels que la jambe-modèle définie par la norme française NF G 30-102b, Annexe B, ou la jambe-modèle type Hohenstein selon le référentiel allemand RAL-GZ 387. Les diverses altitudes correspondantes sont notées conventionnellement *B*, *C* ... *G*.

La règle évoquée plus haut de dégressivité du profil de pression consiste à exercer une pression maximale à la cheville puis dégressive de la cheville au mollet ou à la cuisse. Elle repose sur le fait qu'en situation orthostatique la pression intraveineuse est dégressive de la cheville au mollet puis jusqu'à la cuisse. Dans le cas d'une insuffisance veineuse chronique, la compression élastique exercée par l'orthèse sur le membre va induire un effet anti-stase favorisant le retour veineux.

Le WO 00/01332 A1 (Innothéra Topic International) décrit un exemple de telle structure, en elle-même bien connue, d'orthèse de CVE.

Compte tenu de la nécessité d'appliquer à la cheville la pression maximale, il est indispensable que l'orthèse soit parfaitement ajustée sur le membre à cet endroit.

En général, qu'il s'agisse d'un bas ou d'un collant, l'orthèse est pourvue d'une partie de pied qui empêche dans la région de la cheville tout déplacement de l'orthèse du fait des mouvements naturels, et permet ainsi de conserver l'effet thérapeutique recherché.

Il existe cependant une demande importante de la part des patientes utilisant ce type de produit pour des articles de type caleçon ou *leggings* conformes aux critères actuels de la mode, c'est-à-dire des articles s'arrêtant vers le bas au-dessus de la cheville en laissant le pied découvert, ou même plus haut, s'arrêtant à mi-mollet pour les articles de type "collant 3/4" ou "corsaire".

Le WO 97/45081 A1 (Innothéra Topic International) décrit une orthèse de CVE de type bas ou collant sans pied, pourvue à l'extrémité située au niveau de la cheville un revêtement antidérapant sur la face tournée vers la peau. Ce revêtement vient prendre appui sur les malléoles de la cheville, évitant ainsi tout déplacement de l'orthèse pendant la durée où elle est portée. Le positionnement précis sur la cheville (au niveau des malléoles) laisse toutefois une faible latitude de mise en place. En outre, cette orthèse ne permet pas de découvrir la cheville, ni *a fortiori* une partie du mollet, comme cela serait le cas avec un article de mode de type *leggings.*

Le but de la présente invention est de proposer un article de CVE qui remédie à cette limitation, qui procure un aspect attrayant pour la patiente, similaire à celui des articles de mode de type *leggings* et analogues où la cheville est découverte au-dessus des malléoles jusqu'à un niveau relativement haut, pouvant même laisser apparaître le bas du mollet dans le cas des articles de type "3/4" ou "corsaire".

Le but de l'invention est de pouvoir disposer d'une telle orthèse qui, néanmoins, exerce une CVE sur toute la hauteur du membre, en conformité complète avec les prescriptions réglementaires de définition de ces orthèses, à savoir valeur de pression à la cheville et profil dégressif sur le mollet et sur la cuisse - ceci tout en assurant à la fois un confort et une esthétique comparables à un article de mode de type *leggings* conventionnel.

À cet effet, l'invention propose une orthèse compressive du membre inférieur en forme de bas ou de collant d'un type connu par exemple d'après le WO 97/45081 A1 précité, obtenue par tricotage d'un fil de trame et d'un fil de tricot et comportant une partie compressive s'étendant vers le haut à partir de la cheville jusqu'en haut de cuisse et apte, après que l'orthèse ait été mise en place sur le membre, à exercer en direction circonférentielle une force de rappel élastique propre à produire une compression du membre à un niveau de pression thérapeutique.

De façon caractéristique de l'invention, la partie compressive comprend deux parties distinctes attenantes s'étendant de part et d'autre d'une frontière circonférentielle située à une altitude comprise entre l'altitude du périmètre minimal de la cheville et l'altitude du périmètre maximal du mollet, avec une partie basse relativement transparente, s'étendant depuis la région inférieure de l'orthèse jusqu'à ladite frontière circonférentielle, et une partie haute relativement opaque, s'étendant depuis ladite frontière circonférentielle jusqu'à la région supérieure de l'orthèse. La partie haute est tricotée en continuité avec la partie basse, et la structure de maille et la nature des fils de trame et de tricot sont choisis respectivement pour la partie basse et la partie haute de manière à procurer globalement un profil de pression thérapeutique sensiblement continu, et dégressif à partir de la cheville, sur l'ensemble du membre enveloppé par la partie basse et la partie haute attenante.

Selon diverses caractéristiques subsidiaires avantageuses :
- la transparence de la partie basse relativement transparente est supérieure à 20 %, de préférence supérieure à 25 %, tandis que la transparence de la partie haute relativement opaque est inférieure à 12 %, de préférence inférieure à 10 % ;
- la frontière circonférentielle est située en altitude, à ± 30% près, à mi-distance du périmètre minimal de la cheville et du périmètre maximal du mollet ;
- dans une première forme de réalisation, le fil de tricot de la partie basse et le fil de tricot de la partie haute sont des fils différents, le fil de tricot de la partie basse étant un fil fin et le fil de tricot de la partie haute étant un fil couvrant, tandis que le fil de trame de la partie basse et le fil de trame de la partie haute peuvent être des fils identiques ;
- dans une deuxième forme de réalisation, le fil de trame de la partie basse et le fil de trame de la partie haute sont des fils différents, le fil de trame de la partie basse étant un fil fin et le fil de trame de la partie haute étant un fil couvrant, tandis que le fil de tricot de la partie basse et le fil de tricot de la partie haute peuvent être des fils identiques ;
- dans une troisième forme de réalisation, le fil de tricot de la partie basse et le fil de tricot de la partie haute sont des fils identiques, et la partie haute comprend un fil de tricot supplémentaire par rapport à la partie basse, tandis que le fil de trame de la partie basse et le fil de trame de la partie haute peuvent être des fils identiques ;
- dans une quatrième forme de réalisation, les fils de tricot et de trame de la partie basse sont identiques aux fils de tricot et de trame respectifs de la partie haute, et la hauteur de maille de la partie haute est inférieure à la hauteur de maille de la partie basse ;
- il est prévu au niveau de la frontière circonférentielle une partie de transition séparant la partie basse et la partie haute de l'orthèse et tricotée en continuité avec ces dernières, notamment une partie de transition tricotée en bord-côtes.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue d'ensemble d'une orthèse selon l'invention.
La Figure 2 est une vue en élévation de cette même orthèse, enfilée sur un membre, avec indication des diverses altitudes normalisées auxquelles sont mesurées les pressions appliquées par cette orthèse sur le membre.
La Figure 3 est un diagramme schématique montrant les valeurs de pression relevées à différentes altitudes, pour une orthèse selon l'invention présentant le profil de pression dégressif recherché.

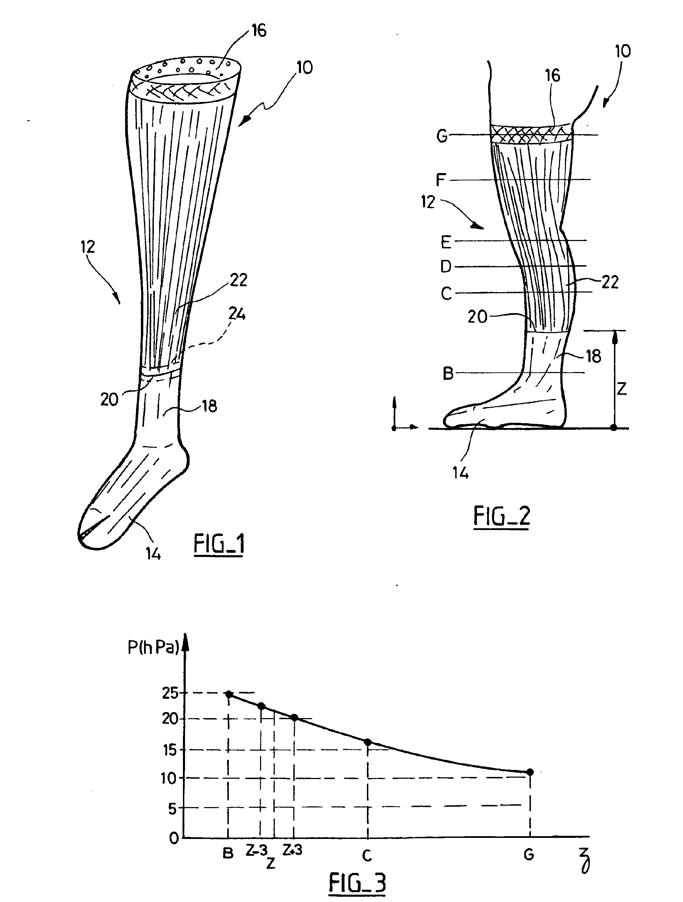

Sur la Figure 1, la référence 10 désigne de façon générale l'orthèse de l'invention, qui est une orthèse tubulaire compressive tricotée dont la structure de maille et le dimensionnement sont choisis de manière à appliquer sur le membre élastique une compression élastique une fois l'orthèse enfilée sur le membre. Cette orthèse comprend une partie compressive 12 enveloppant la cheville et le mollet, terminée en partie inférieure par une partie de pied 14. En partie supérieure, la partie compressive 12 s'étend sur la hauteur de la cuisse et comporte à son extrémité supérieure une bande 16 pourvue intérieurement de motifs antidérapants, par exemple en silicone, destinés à assurer le maintien en place sur le membre sans risque de glissement.

Cette configuration de "bas-cuisse" n'est cependant pas limitative de l'invention, qui peut être également réalisée sous forme d'un collant.

Sur la figure 2, on a représenté les diverses altitudes du membre inférieur telles que définies par le modèle Hohenstein de jambe de référence :

| | |
|---|---|
| *B :* | au périmètre minimal de la cheville, soit à une altitude *z* = 12 cm par rapport au sol, |
| *C* : | au périmètre maximal du mollet, à *z* = 31 cm, |
| *D* : | au-dessous du genou, à *z* = 39 cm, |
| *E* : | au creux poplité, à *z* = 45 cm, |
| *F* : | en milieu de cuisse, à *z* = 60 cm, |
| *G* : | en haut de cuisse, à *z* = 72 cm. |

Dans le cas des orthèses conventionnelles, la pression exercée à l'altitude du point B du modèle Hohenstein, c'est-à-dire au niveau de la cheville, est la pression prescrite pour la classe de contention/compression choisie (I, II, III ou IV) cette pression correspondant par ailleurs à la valeur de pression théoriquement la plus élevée exercée sur le membre.

On prendra ici l'exemple d'une orthèse de classe Il, qui est la classe la plus couramment prescrite pour les bas ou collants de CVE. Bien entendu, cet exemple n'est pas limitatif, et il peut être décliné, *mutatis mutandis,* pour des profils de pression d'autres classes.

Le profil de pression est défini à partir de valeurs de pression produite localement par l'orthèse mesurées sur un gabarit de référence, valeurs qui peuvent être relevées de manière classique, par exemple sur dynamomètre conformément à la norme NF G30 102 - B).

Pour respecter les prescriptions réglementaires, l'orthèse doit respecter, pour la classe 11, les conditions suivantes :
- pression P_{B} au niveau de la cheville (à l'altitude B) comprise entre 15 et 20 mm Hg,
- dégressivité d'au moins 70 % en haut de cuisse, c'est-à-dire pression P_{G} en haut de cuisse (à l'altitude G) inférieure à 70 % de la pression P_{B} à la cheville.

La caractéristique essentielle de l'invention consiste à réaliser la partie compressive 12 d'une telle orthèse en deux parties distinctes 18, 22 avec :
- une partie basse transparente 18 laissant voir la peau, s'étendant du pied jusqu'à une frontière circonférentielle 20 située approximativement à mi-mollet, et
- une partie haute opaque 22 masquant la peau, allant de la frontière circonférentielle 20 à mi-mollet jusqu'au haut de l'orthèse.

Par "transparente" et "opaque" on entendra une transparence et une opacité relatives, c'est-à-dire que la partie basse 18 présente une transparence supérieure (ou, en d'autres termes, une opacité inférieure) à celle de la partie haute 22.

La transparence d'un article textile extensible peut être quantifiée de manière précise, notamment par le procédé du WO 2006/051183 A1 (Innothéra Topic International), qui décrit un procédé et un appareillage de mesure de la transparence d'un article textile extensible dans un état de tension correspondant à l'état où il se trouve lorsqu'il est enfilé sur un membre, donc en reflétant le résultat final, au porté, de l'article une fois enfilé par le patient.

De préférence, la partie basse 18 présente une transparence supérieure à 20 %, de préférence supérieure à 25 %, et la partie haute 22 une transparence inférieure à 12 %, de préférence inférieure à 10 %, ces valeurs étant déterminées suivant la méthode de mesure décrite dans le WO 2006/051183 A1 précité.

Une première caractéristique importante de l'invention est que la partie haute opaque 22 est tricotée en continuité avec la partie basse transparente 18. En d'autres termes, il ne s'agit pas d'une partie rapportée, mais d'une partie tricotée au cours d'une même séquence sur la machine de tricotage : au passage de la frontière 20 entre les deux parties, il y aura seulement changement du fil de tricot, et/ou du fil de trame, et/ou de la hauteur de maille, et/ou ajout d'un fil de tricot (ces différentes possibilités seront exposées en détail plus bas), ceci en continuité au cours de la même séquence de tricotage.

Une autre caractéristique importante, et essentielle, de l'invention est que, malgré cette transition impliquant un changement de fil(s) et/ou de paramètre(s) de tricotage, le profil de pression appliqué le long du membre reste sensiblement continu, avec une dégressivité à partir de la cheville en remontant sur le mollet et jusqu'en haut de la cuisse. Les réglages de la machine à tricoter seront à cet égard adaptés de manière que, du seul point de vue des pressions appliquées par l'orthèse, la transition de part et d'autre de la zone frontière 20 se fasse de la façon la plus continue possible, comme avec un bas-cuisse conventionnel qui serait réalisé avec une structure et un tricotage homogènes.

Ainsi, sur la Figure 3 on a illustré le profil de pression relevé pour une orthèse selon l'invention, et l'on peut voir que les prescriptions réglementaires en matière de pression exercée à la cheville et de dégressivité sont parfaitement respectées malgré l'hétérogénéité de texture entre la partie basse transparente 18 (altitude z en-deçà du point Z) et la partie haute opaque 22 (*z* au-delà du point Z). La figure indique notamment les pressions aux altitudes B (au périmètre minimal de la cheville) et C (au périmètre maximal du mollet) et G (en haut de cuisse), montrant une transition régulière du profil de pression sur toute la longueur du membre inférieur, notamment sur toute la partie située au-dessous du genou.

Plus précisément, les valeurs suivantes de pression ont été mesurées :

| | |
|---|---|
| Point B : | 23,5 hPa (soit 17,4 mmHg) ; |
| Point Z-3 cm (zone transparente) : | 22 hPa (soit 16,3 mmHg) ; |
| Point Z+3 cm (zone opaque) : | 20,6 hPa (soit 15,3 mmHg) ; |
| Point C : | 15,5 hPa (soit 11,5 mm Hg) ; |
| Point G : | 12,1 hPa (soit 9 mmHg). |

On notera que la transition entre la partie basse 18 et la partie haute 22 au niveau de la frontière circonférentielle 20 peut être soit une transition franche, soit une transition progressive atténuée par un tricotage particulier dans une partie de transition 24 autour de la frontière circonférentielle. Cette transition progressive peut résulter de l'utilisation d'un jeu de mailles particulier, par exemple avec un tricotage donnant une apparence "bord-côtes" à l'extrémité inférieure de la partie haute opaque 22.

On va maintenant décrire plusieurs exemples de structures permettant d'obtenir le résultat recherché, à savoir l'obtention d'un effet *leggings* sur un produit de CVE, avec la garantie pour la patiente d'une parfaite continuité de la dégressivité tout au long de la jambe.

Chacun des exemples ci-dessous décrit une technique particulière (changement du fil de tricot sans changement du fil de trame, changement du fil de trame sans changement du fil de tricot, etc.) permettant d'obtenir le résultat recherché, mais ces techniques ne sont pas exclusives l'une de l'autre et peuvent être combinées entre elles (par exemple changement à la fois du fil de tricot et du fil de trame, etc.).

### Exemple 1

Une première technique consiste à conserver le même fil de trame pour le tricotage de l'orthèse sur la partie basse transparente 18 et la partie haute opaque 22, mais à changer le fil de tricot, en choisissant un fil fin pour la partie basse 18 et un fil couvrant pour la partie haute 22. On entendra par "fil fin" un fil qui ne remplit pas la maille tricotée et laisse donc voir la peau au travers de la maille, et par "fil couvrant" un fil qui remplit la maille (du fait de son titre, de sa texturation, du nombre de brins utilisés, etc.) et qui ne laisse donc pas, ou peu, voir la peau au travers de la maille.

Les fils suivants peuvent notamment être utilisés à cet effet :
- fil de tricot de la partie basse : élasthanne 17 dtex simple guipé avec du PA 1/42/46,
- fil de tricot de la partie haute : élasthanne 17 dtex simple guipé avec du PA 1/85/92,
- fil de trame unique sur l'ensemble de la jambe: élasthanne 330 dtex double guipé avec du PA 1/22/7.

### Exemple 2

Une deuxième technique consiste à conserver le même fil de tricot pour les deux parties, en changeant le fil de trame de la partie basse 18 à la partie haute 22. Le changement du fil de trame peut être obtenu par croisement de fils : lors de l'opération de tricotage, le fil tramé de la partie haute 22 est progressivement interrompu, et le nouveau fil de trame est progressivement engagé, les deux fils se chevauchant sur quelques colonnes de mailles.

Les fils suivants peuvent notamment être utilisés à cet effet :
- fil de trame de la partie basse : élasthanne 310 dtex double guipé avec du PA 1 /22/7,
- fil de trame de la partie haute : élasthanne 310 dtex double guipé avec du PA 1/60/68,
- fil de tricot unique sur l'ensemble de la jambe : élasthanne 17 dtex simple guipé avec du PA 1/42/46.

### Exemple 3

Une troisième technique consiste à garder les mêmes fils de trame et de tricot sur toute la hauteur de la jambe, en ajoutant un fil de tricot supplémentaire au niveau de la partie haute 22. En d'autres termes, lors du tricotage de la partie haute 22, deux fils de tricot sont utilisés en parallèle, et un seul de ces deux fils subsiste lors du tricotage de la partie basse 18.

Les fils suivants peuvent notamment être utilisés à cet effet :
- fil de trame unique sur l'ensemble de la jambe : élasthanne 330 dtex double guipé avec du PA 1/22/7,
- fil de tricot unique sur l'ensemble de la jambe : élasthanne 22 dtex double guipé avec du PA 1/22/7,
- fil de tricot ajouté en partie haute : élasthanne 22 dtex double guipé avec du PA 1/44/34.

### Exemple 4

Une quatrième technique consiste à modifier la hauteur de maille entre les deux parties, avec une maille "aérée" (plus lâche) pour la partie basse 18 et une maille plus serrée pour la partie haute 22. Le changement de la hauteur de maille est obtenu par une variation de la longueur de fil absorbée (LFA) lors de l'opération de tricotage.

Les fils et paramètres suivants peuvent notamment être utilisés à cet effet :
- fil de trame unique sur l'ensemble de la jambe : élasthanne 310 dtex double guipé avec du PA 1/22/7,
- fil de tricot unique sur l'ensemble de la jambe : élasthanne 17 dtex simple guipé avec du PA 1/42/46,
- hauteur de maille en partie basse deux fois plus importante que la hauteur de maille de la partie haute.

## Revendications

1. Une orthèse compressive du membre inférieur en forme de bas ou de collant,
cette orthèse (10) étant obtenue par tricotage d'un fil de trame et d'un fil de tricot et comportant une partie compressive (12) s'étendant vers le haut à partir de la cheville (B) jusqu'en haut de cuisse (G) et apte, après que l'orthèse ait été mise en place sur le membre, à exercer en direction circonférentielle une force de rappel élastique propre à produire une compression du membre à un niveau de pression thérapeutique,
cette orthèse étant **caractérisée en ce que** :
- la partie compressive (12) comprend deux parties distinctes attenantes (18, 22) s'étendant de part et d'autre d'une frontière circonférentielle (20) située à une altitude (Z) comprise entre l'altitude (B) du périmètre minimal de la cheville et l'altitude (C) du périmètre maximal du mollet, avec :
• une partie basse (18) relativement transparente, s'étendant depuis la région inférieure de l'orthèse (14) jusqu'à ladite frontière circonférentielle (20), et
• une partie haute (22) relativement opaque, s'étendant depuis ladite frontière circonférentielle (20) jusqu'à la région supérieure de l'orthèse (16) ;
- la partie haute est tricotée en continuité avec la partie basse ; et
- la structure de maille et la nature des fils de trame et de tricot sont choisis respectivement pour la partie basse et la partie haute de manière à procurer globalement un profil de pression thérapeutique sensiblement continu, et dégressif à partir de la cheville, sur l'ensemble du membre enveloppé par la partie basse et la partie haute attenante.

2. L'orthèse de la revendication 1, dans laquelle la transparence de la partie basse relativement transparente est supérieure à 20 %, de préférence supérieure à 25 %.

3. L'orthèse de la revendication 1, dans laquelle la transparence de la partie haute relativement opaque est inférieure à 12 %, de préférence inférieure à 10 %.

4. L'orthèse de la revendication 1, dans laquelle la frontière circonférentielle (20) est située en altitude (Z), à ± 30% près, à mi-distance du périmètre minimal de la cheville (B) et du périmètre maximal du mollet (C).

5. L'orthèse de la revendication 1, dans laquelle le fil de tricot de la partie basse et le fil de tricot de la partie haute sont des fils différents, le fil de tricot de la partie basse étant un fil fin et le fil de tricot de la partie haute étant un fil couvrant.

6. L'orthèse de la revendication 5, dans laquelle le fil de trame de la partie basse et le fil de trame de la partie haute sont des fils identiques.

7. L'orthèse de la revendication 1, dans laquelle le fil de trame de la partie basse et le fil de trame de la partie haute sont des fils différents, le fil de trame de la partie basse étant un fil fin et le fil de trame de la partie haute étant un fil couvrant.

8. L'orthèse de la revendication 7, dans laquelle le fil de tricot de la partie basse et le fil de tricot de la partie haute sont des fils identiques.

9. L'orthèse de la revendication 1, dans laquelle le fil de tricot de la partie basse et le fil de tricot de la partie haute sont des fils identiques, et la partie haute comprend un fil de tricot supplémentaire par rapport à la partie basse.

10. L'orthèse de la revendication 9, dans laquelle le fil de trame de la partie basse et le fil de trame de la partie haute sont des fils identiques.

11. L'orthèse de la revendication 1, dans laquelle les fils de tricot et de trame de la partie basse sont identiques aux fils de tricot et de trame respectifs de la partie haute, et la hauteur de maille de la partie haute est inférieure à la hauteur de maille de la partie basse.

12. L'orthèse de la revendication 1, comprenant au niveau de la frontière circonférentielle (20) une partie de transition (24) séparant la partie basse (18) et la partie haute (22) de l'orthèse, et tricotée en continuité avec ces dernières.

13. L'orthèse de la revendication 12, dans laquelle la partie de transition (24) est une partie tricotée en bord-côtes.

## Claims

1. A compressive orthosis for the lower limb in the form of stockings or a pair of tights
said orthosis (10) being obtained by knitting a weft yarn and a knitting yarn and comprising a compressive portion (12) extending upward from the ankle (B) as far as the top of the thigh (G) and capable, after the orthosis has been placed in position on the limb, of exerting an elastic restoring force in the circumferential direction appropriate for producing compression of the limb at a therapeutic amount of pressure, said orthosis being **characterized in that**:
- the compressive portion (12) includes two distinct adjoining parts (18, 22) extending on both sides of a circumferential border (20) located at a height (Z) between the height (B) of the minimum perimeter of the ankle and the height (C) of the maximum perimeter of the calf inclusive, with:
- a relatively transparent bottom part (18), extending from the lower region of the orthosis (14) as far as said circumferential border (20), and
- a relatively opaque top part (22), extending from said circumferential border (20) as far as the upper region of the orthosis (16):
- the top part is knitted continuously with the bottom part; and
- the mesh structure and the nature of the weft and knitting yarns are selected for the bottom part and the top part respectively so as to provide overall a profile of appreciably continued therapeutic pressure, which becomes less from the ankle, over the whole of the limb enveloped by the bottom part and the adjacent top part.

2. The orthosis of claim 1, in which the transparency of the relatively transparent bottom part is in excess of 20%, preferably in excess of 25%.

3. The orthosis of claim 1, in which the transparency of the relatively opaque top part is less than 12%, preferably less than 10%.

4. The orthosis of claim 1, in which the circumferential border (20) is located at height (Z), approximately ± 30%, halfway between the minimum perimeter of the ankle (B) and of the maximum perimeter of the calf (C).

5. The orthosis of claim 1, in which the knitting yarn of the bottom part and the knitting yarn of the top part are different yarns, the knitting yarn of the bottom part being a fine yarn and the knitting yarn of the top part being a covering yarn.

6. The orthosis of claim 5, in which the weft yarn of the bottom part and the weft yarn of the top part are identical yarns.

7. The orthosis of claim 1, in which the weft yarn of the bottom part and the weft yarn of the top part are different yarns, the weft yarn of the bottom part being a fine yarn and the weft yarn of the top part being a covering yarn.

8. The orthosis of claim 7, in which the knitting yarn of the bottom part and the knitting yarn of the top part are identical yarns.

9. The orthosis of claim 1, in which the knitting yarn of the bottom part and the knitting yarn of the top part are identical yarns, and the top part includes an additional knitting yarn compared with the bottom part.

10. The orthosis of claim 9, in which the weft yarn of the bottom part and the weft yarn of the top part are identical yarns.

11. The orthosis of claim 1, in which the knitting and weft yarns of the bottom part are identical to the respective knitting and weft yarns of the top part, and the height of the mesh of the top part is less than the height of the mesh of the bottom part.

12. The orthosis of claim 1, including a transition part (24) at the circumferential border (20) separating the bottom part (18) and the top part (22) of the orthosis, knitted continuously with said bottom and top parts.

13. The orthosis of claim 12, in which the transition part (24) is a part that has been rib knitted.

## Patentansprüche

1. Kompressionsorthese der unteren Gliedmaße in Form eines Strumpfs oder einer Strumpfhose,
wobei diese Orthese (10) durch Stricken eines Schussfadens und eines Strickfadens erhalten wird und einen Kompressionsteil (12) aufweist, der sich vom Knöchel (B) ausgehend bis zum Oberschenkel (G) erstreckt und, nachdem die Orthese auf dem Glied angeordnet wurde, in Umfangsrichtung eine elastische Rückstellkraft ausüben kann, die eine Komprimierung der Gliedmaße auf einem therapeutischen Druckpegel erzeugen kann,
wobei diese Orthese **dadurch gekennzeichnet ist, dass**:
- der Kompressionsteil (12) zwei getrennte angrenzende Teile (18, 22) enthält, die sich zu beiden Seiten einer Umfangsgrenze (20) erstrecken, welche sich auf einer Höhe (Z) befindet, die zwischen der Höhe (B) des minimalen Umfangs des Knöchels und der Höhe (C) des maximalen Umfangs der Wade liegt, mit:
• einem relativ durchsichtigen unteren Teil (18), der sich vom unteren Bereich der Orthese (14) bis zur Umfangsgrenze (20) erstreckt, und
• einem relativ undurchsichtigen oberen Teil (22), der sich von der Umfangsgrenze (20) bis zum oberen Bereich der Orthese (16) erstreckt;
- der obere Teil in Kontinuität mit dem unteren Teil gestrickt ist; und
- die Maschenstruktur und die Beschaffenheit des Schussfadens und des Strickfadens für den unteren Teil bzw. den oberen Teil so gewählt werden, dass sie global ein im Wesentlichen durchgehendes, und ausgehend vom Knöchel abnehmendes, therapeutisches Druckprofil über die Gesamtheit der vom unteren Teil und vom daran angrenzenden oberen Teil umhüllten Gliedmaße liefern.

2. Orthese nach Anspruch 1, bei der die Durchsichtigkeit des relativ durchsichtigen unteren Teils größer als 20 %, vorzugsweise größer als 25 % ist.

3. Orthese nach Anspruch 1, bei der die Durchsichtigkeit des relativ undurchsichtigen oberen Teils geringer als 12 %, vorzugsweise geringer als 10 % ist.

4. Orthese nach Anspruch 1, bei der die Umfangsgrenze (20) sich in der Höhe (Z), bis auf ± 30 %, auf halber Strecke zwischen dem minimalen Umfang des Knöchels (B) und dem maximalen Umfang der Wade (C) befindet.

5. Orthese nach Anspruch 1, bei der der Strickfaden des unteren Teils und der Strickfaden des oberen Teils unterschiedliche Fäden sind, wobei der Strickfaden des unteren Teils ein feiner Faden und der Strickfaden des oberen Teils ein deckender Faden ist.

6. Orthese nach Anspruch 5, bei der der Schussfaden des unteren Teils und der Schussfaden des oberen Teils gleiche Fäden sind.

7. Orthese nach Anspruch 1, bei der der Schussfaden des unteren Teils und der Schussfaden des oberen Teils unterschiedliche Fäden sind, wobei der Schussfaden des unteren Teils ein feiner Faden und der Schussfaden des oberen Teils ein deckender Faden ist.

8. Orthese nach Anspruch 7, bei der der Strickfaden des unteren Teils und der Strickfaden des oberen Teils gleiche Fäden sind.

9. Orthese nach Anspruch 1, bei der der Strickfaden des unteren Teils und der Strickfaden des oberen Teils gleiche Fäden sind, und der obere Teil einen zusätzlichen Strickfaden bezüglich des unteren Teils enthält.

10. Orthese nach Anspruch 9, bei der der Schussfaden des unteren Teils und der Schussfaden des oberen Teils gleiche Fäden sind.

11. Orthese nach Anspruch 1, bei der die Strick- und Schussfäden des unteren Teils gleich den Strick- und Schussfäden des oberen Teils sind, und die Maschenhöhe des oberen Teils kleiner als die Maschenhöhe des unteren Teils ist.

12. Orthese nach Anspruch 1, die im Bereich der Umfangsgrenze (20) einen Übergangsteil (24) enthält, der den unteren Teil (18) und den oberen Teil (22) der Orthese trennt und in Kontinuität mit diesen gestrickt ist.

13. Orthese nach Anspruch 12, bei der der Übergangsteil (24) ein als Strickbündchen gestrickter Teil ist.
